(19) Europäisches Patentamt
European Patent Office
Office européen des brevets

(11) **EP 4 374 771 A1**

(12) **EUROPEAN PATENT APPLICATION**

(43) Date of publication:
**29.05.2024 Bulletin 2024/22**

(21) Application number: **22383130.6**

(22) Date of filing: **24.11.2022**

(51) International Patent Classification (IPC):
***A61B 3/10*** (2006.01)    ***G01B 9/02*** (2022.01)
***G02B 6/28*** (2006.01)    ***G02B 26/00*** (2006.01)

(52) Cooperative Patent Classification (CPC):
**A61B 3/1005; G01B 9/02; G02B 26/105**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **2Eyes Vision, S.L.**
**28760 Tres Cantos (ES)**

(72) Inventors:
• **CURATOLO, Andrea**
  **Warsaw (PL)**
• **URIZAR URSUA, María Pilar**
  **Tres Cantos (ES)**
• **GAMBRA URRALBURU, Enrique**
  **Tres Cantos (ES)**

(74) Representative: **Elion IP, S.L.**
**Paseo Castellana, 150-4 dcha**
**28046 Madrid (ES)**

(54) **OPTICAL DELAY LINE, INTERFEROMETER AND METHOD FOR GENERATING AN OPTICAL DELAY BASED ON A SPINNING TILTED MIRROR**

(57)    The present disclosure relates to an optical delay line (100, 101, 102), comprising a plurality of optical elements in optical communication with each other, wherein:
- at least one of said plurality of optical elements is a diffractive element (10),
- at least one of said plurality of optical elements is a reflective element (30),
- at least one of said plurality of optical elements is a refractive element (20), the refractive element (20) being located between the diffractive element (10) and the reflective element (30);
wherein the reflective element (30) is tilted at an angle ($\alpha$, $\alpha'$, $\alpha''$) with respect to a plane (53), the reflective element (30) being rotatable around a rotation axis (81, 52) while the angle ($\alpha$, $\alpha'$, $\alpha''$) is maintained, the plane (53) being orthogonal to the rotation axis (81, 52).

The disclosure also relates to an interferometer comprising such an optical delay line, and also to a method for generating an optical delay.

FIG. 4

EP 4 374 771 A1

## Description

The present disclosure relates to the field of optical measurements, in particular to optical measurement using frequency domain rapid scanning optical delay lines. The present disclosure has application in the field of partial coherence interferometry and optical coherence tomography.

## BACKGROUND

Optical biometers are used to perform ocular biometry (also known as optical or ophthalmic biometry), which refers to measuring ocular biometric parameters, such as the eye axial length (~25 mm), the eye anterior chamber depth (~ 4 mm) and the corneal thickness (~ 0.5 mm). Currently, commercial optical biometers have largely replaced ultrasound based biometers because of their higher resolution, non-invasiveness and repeatability. However, these optical biometers are significantly more expensive (>$20.000) and they are benchtop devices, i.e. vibration adverse and not-particularly compact.

Optical biometers measure the ocular biometric parameters by scanning the optical group delay generated in an interferometric setup between its reference and sample arm, using low-temporal coherence infrared light sources. Different interferometric setups are used in the aforementioned biometers, namely, for example, Partial Coherence Interferometry (PCI) and Optical Low Coherence Reflectometry (OLCR) setups.

A simple way to scan the optical group delay is by inducing an optical path length difference with an optical delay line in an arm of the interferometer, commonly named the reference arm. A standard optical delay line is an axially moving mirror upon which an optical beam is incident. Another possibility is by using a rotating glass cube system. These techniques are the most commonly employed in the previously mentioned commercial biometers.

However, there are some disadvantages in these optical delay lines for measuring ocular biometric parameters, these disadvantages essentially being:

- the mirror needs to be displaced a large distance in order to scan the required range of optical group delay to measure the axial length required for an optical biometry measurement; and
- limited scan-speeds and vibration adversity due to moving parts over substantial distances.

To increase the optical delay induced, other techniques have been proposed, using integrated optics; multiple reference paths; galvanometer and MEMS mirrors; moving-coil actuators; frequency-domain rapid scanning optical delay lines; and rotating prisms and polygon mirrors.

Among all of them, frequency-domain optical delay lines (FDODL) have been used in time domain OCT (TD-OCT) mainly for rapid and long-range scanning since, in order to generate an axial scan, they only involve a tilt of a small mirror back and forth over a short angular range, typically implemented with a galvanometric scanner. Thus, FDODL would also be suited for robust long-range scanning in ocular biometry. Patent document US-6111645-A discloses an apparatus achieving optical delay scanning by using diffractive optical elements in conjunction with a galvanometric mirror and imaging optics. This apparatus permits the optical group delay to be scanned by scanning the angle of the galvanometric mirror. However, the high cost of a galvanometric mirror scanner makes its use impractical for low-cost applications.

Thus, there is still a need for a low-cost, compact and robust optical delay line avoiding the use of moving parts over large distances and affordable for the end user.

## SUMMARY

The present disclosure solves the shortcomings of the existing solutions with a novel optical delay line, robust and simple in construction, which can be provided at low-cost.

An aspect of this disclosure to an optical delay line comprises a plurality of optical elements in optical communication with other, wherein:

- at least one of said plurality of optical elements is a diffractive element,
- at least one of said plurality of optical elements is a reflective element,
- at least one of said plurality of optical elements is a refractive element, the refractive element being located between the diffractive element and the reflective element.

According to this aspect, the reflective element is tilted at an angle with respect to a plane, the reflective element being rotatable around a rotation axis while the angle of tilt is maintained, the plane being orthogonal to the rotation axis.

By maintaining a constant angle of tilt of the reflective element when the reflective element is rotated, the reflective element acts as a scanning component. Thus, a variation of the optical path length of an input light beam inputted into the optical delay line is generated during rotation of the tilted reflective element.

That is, when the optical delay line is in use, and the reflective element is rotated, the angle of tilt or inclination of the reflective element with respect to the plane orthogonal to the axis of rotation is maintained fixed during the rotation.

The rotation axis of the reflective element can be collinear or at a specific fixed angle with the optical axis of the refractive element. An advantage of the collinear configuration is that the optical delay line can be manufactured in a simple and robust fashion.

In certain embodiments a centre of rotation of the reflective element has an offset distance with respect to the intersection of the optical axis of the refractive element with the surface of the rotating reflective element. The presence of an offset allows to implement a configuration where the rotation axis is parallel to the optical axis of the refractive element still allowing for a simple configuration.

The diffractive element can be implemented as a diffraction grating. The diffractive element can be arranged to receive an input light beam entering the optical delay line along an input axis, the input axis being at an input angle with respect to optical axis of the refractive element. The input angle is such that allows to avoid the Littrow configuration of the diffractive element used. The input axis can also be arranged at an input offset distance, in the plane of the diffractive element, with respect to the optical axis of the refractive element. A preferred configuration for the input offset distance at the diffractive element between the input axis and the optical axis of the refractive element is such that is equal to zero. A preferred configuration for the input angle is such that a central wavelength of the received input light beam along the input axis, after a first incidence on the diffractive element, is directed collinear to the optical axis of the refractive element. The diffractive element can also be arranged with the direction of its grooves perpendicular to the optical axis of the refractive element.

Thus, when the optical delay line is in use, and the reflective element is rotated while maintaining fixed the angle of tilt of the reflective element with respect to the plane orthogonal to the axis of rotation, the reflected light beam is directed towards a second incidence on the diffractive element being parallelly displaced with respect to the input axis at the diffractive element, varying at least its position along the orthogonal direction of the grooves of the diffractive element with the rotation of the tilted reflected element.

In certain embodiments the reflective element is a mirror. Other examples of the reflective mirror include a beam splitter, a prism, a glass, or any other flat surface with a reflective coefficient. The reflective element can be arranged to reflect at least a portion of the input light beam diffracted by the diffractive element and refracted by the refractive element.

Examples of the refractive element are a lens, a group of lenses, a lens system, a concave mirror and a spatial light modulator. In certain embodiments the lens is an achromatic double lens, which reduces chromatic aberration. This refractive element can be arranged to receive at least a portion of a collimated and dispersed light beam produced by the diffractive element upon receiving an input light beam. This refractive element can be arranged at a distance equal to its focal length from the intersection of the input axis at the diffractive element, as well as at a distance equal to its focal length to the reflective element. Having the diffractive element and the reflective element at a focal distance provides a simple configuration while guaranteeing optimal performance of the optical delay line.

The angle of tilt of the reflective element may be adjusted through a range of degrees. This way, the range of optical path length variation of the optical delay line can be adjusted and selected to provide different scanning ranges. In certain embodiments, the angle of tilt is more than 0° and less than 10°, which allows using standard optical elements; for instance, a standard lens having a diameter of around 5 cm (2 inches) and a focal length of around 75 mm can be used as refractive element.

In certain embodiments, the specific fixed angle between the rotation axis of the reflective element and the optical axis of the refractive element is smaller than 90 degrees, and is preferably kept smaller than 10 degrees. This allows the dispersed and refracted input light beam to be incident on the reflective element, as well to allow the sequentially reflected beam to pass through the refractive element again. The offset between the centre of rotation and the optical axis of the refractive element may be at least smaller than half of the size of the reflective element in the direction of the offset. The offset can be less than 3.5 mm when a small mirror of 7 mm of diameter is used as reflective element, and when the centre of rotation is placed at the centre thereof.

In certain embodiments the optical delay line further comprises a motor. The reflective element can be made rotatable with respect to or parallelly to the rotation axis by means of this motor. This way, by varying a speed of the motor, the optical delay line can provide different scanning frequencies. The motor can be coupled to the reflective element.

Certain embodiments comprise a stepper motor or a DC motor, which are widely provided at low-cost, and are easy to couple to the reflective element. Usually, an output shaft of the motor is made parallel to the optical axis of the optical delay apparatus. In a simple and robust configuration, an output shaft of the motor is made collinear with the rotation axis of the reflective element. In this latter configuration, an outermost end of the shaft of the motor can be arranged to be the reflective element.

Some embodiments of the optical delay line further comprise at least another reflective element which is arranged perpendicular to the input axis along which an input light beam entering the optical delay line, and sequentially after a second incidence of the input light beam in the diffractive element.

That is, a further reflective element can be provided, which is arranged sequentially after a second incidence on the diffractive element, and it is perpendicular to the input axis. This further reflective element may comprise a through hole, so as to enable a light beam to go through it. This through hole is typically centred within the further reflective element.

Another aspect of this disclosure relates to an interferometer, the interferometer comprising at least a first arm and a second arm, wherein the first arm is arranged in optical communication with an optical delay line as defined in the foregoing.

The interferometer is typically configured for splitting light coming from an optical source, such as an SLED, into at least the first and the second arms.

In some embodiments, the interferometer further comprises at least a detector, which is arranged such that the light coming from the optical source, after propagation through the first and second arms, interferes at the detector, as in Optical Low Coherence Reflectometry (OLCR) wherein the first arm is the reference arm and the second arm is the sample arm.

In some embodiments, the interferometer comprises at least a third arm arranged in optical communication with a reflective element which is arranged such that the light, after propagation through the first arm and the third arm, is arranged in optical communication with the second arm, acting as a sample arm. The interferometer typically comprises at least a detector which is arranged such that light coming from the optical source, after propagation through the first arm, the third arm and the second or sample arm, interferes at the detector, as in partial coherence interferometry (PCI) using a dual-beam interferometer setup.

In some embodiments, the optical delay line is implemented as a frequency-domain optical delay line typically used in PCI interferometers.

In such configurations where the optical delay line is arranged in optical communication with the first or reference arm of an interferometer, a phase modulator may be added in the first arm of the interferometer if the centre of rotation of the reflective element is on the optical axis of the refractive element, thus being the offset between the optical axis of the refractive element and the centre of rotation of the reflective element equal to zero. The phase modulator allowing in such cases to generate the carrier frequency of the interferometric signal of the PCI interferometer. A preferred configuration of the optical delay line as defined in the foregoing when used in optical communication with the reference arm of an interferometer is such that the offset distance between the centre of rotation of the reflective element and the optical axis of the refractive element is different to zero, avoiding the use of a phase modulator.

Another aspect of the disclosure relates to a method for generating an optical delay, the method comprising steps of:

- receiving an input light beam;
- diffracting the input light beam into a dispersed light beam;
- refracting the dispersed light beam into a focused light beam;
- reflecting, by a reflective element, at least a portion of the focused light beam; wherein the step of reflecting is carried out at different angular positions of the reflective element upon rotating the reflective element around a rotation axis.

Upon rotating the reflective element, the output light beam is spatially displaced with respect to the input axis at each angular position of rotation of the reflective element, and thus having travelled different optical path lengths at each angular positions and having generated an optical delay for the input light beam between each angular position.

In certain embodiments the reflective element is tilted at a fixed angle with respect to a plane orthogonal to a rotation axis. By maintaining the angle of tilt of the reflective element fixed during the rotation of the reflective element, the reflective element acts as a scanning component due to the variation of a reflected input light beam position along the axis orthogonal to the direction of the grooves of the diffraction grating.

In some embodiments, the input light beam to the optical delay line is subject to phase modulation.

The input light beam can be received at an input angle, the input angle being different to zero, prior to its diffraction into a dispersed light beam.

The different aspects and embodiments defined in the foregoing may be combined with one another, as long as they are compatible with each other.

Additional advantages and features of the present disclosure will become apparent from the detail description that follows and will be particularly pointed out in the appended claims.

## BRIEF DESCRIPTION OF THE DRAWINGS

To complete the description and in order to provide for a better understanding of the present disclosure, a set of drawings is provided. Said drawings form an integral part of the description and illustrate one or more embodiments, which should not be interpreted as restricting the scope of the disclosure, but just as an example of how the present disclosure can be carried out. The drawings comprise the following figures:

Figure 1 is a schematic top view of a standard grating-based optical delay line in a single pass configuration using as the scanning component a variable tilt mirror, such as a galvanometric mirror, around the Y axis.

Figure 2 is a schematic perspective view of the standard grating-based optical delay line of Figure 1, showing the effect of the change in the angle of the galvanometric mirror on the output light beam.

Figures 3A and 3B are schematic top views of the proposed grating-based optical delay line in a single pass configuration, using a spinning tilted mirror as scanning component. Each of Figures 3A and 3B shows a different rotational position of the spinning tilted mirror, which has been rotated 180 degrees around the Z axis between each other.

Figure 4 is a schematic perspective view of the proposed grating-based optical delay line shown in Figures 3A-3B, showing the effect on the output light beam of rotating the spinning tilted mirror around the optical axis on the output light beam.

Figure 5A is a schematic perspective view of an optical delay line, in which the spinning tilted mirror is set at three different tilted angles.

Figure 5B shows the optical pathlength variation induced during the displacements of the output light beam of the three options shown in Figure 5A when the spinning tilted mirror is rotated.

Figures 6A and 6B show schematic top views of a proposed grating-based optical delay line in a double-pass configuration, including a perforated double-pass mirror. Each of Figures 6A and 6B shows a different rotational position of the spinning tilted mirror, which has been rotated 180 degrees around the Z axis between each other.

Figure 7A schematically shows an interferometer in an OLCR configuration where the optical delay line shown in Figures 6A and 6B is included in optical communication with a first arm of the OLCR interferometer.

Figure 7B schematically shows an interferometer in a dual-beam PCI interferometer where the optical delay line shown in Figures 6A and 6B is included in one of the arms.

Figures 8A and 8B are schematic top views of another grating-based optical delay line, wherein the axis of rotation of the spinning tilted mirror is tilted with respect to the optical axis of the refractive element. Similarly, to Figures 3A-3B, these figures show two different rotational positions of the spinning tilted mirror, there being 180° between them.

## DESCRIPTION OF PREFERRED EMBODIMENTS

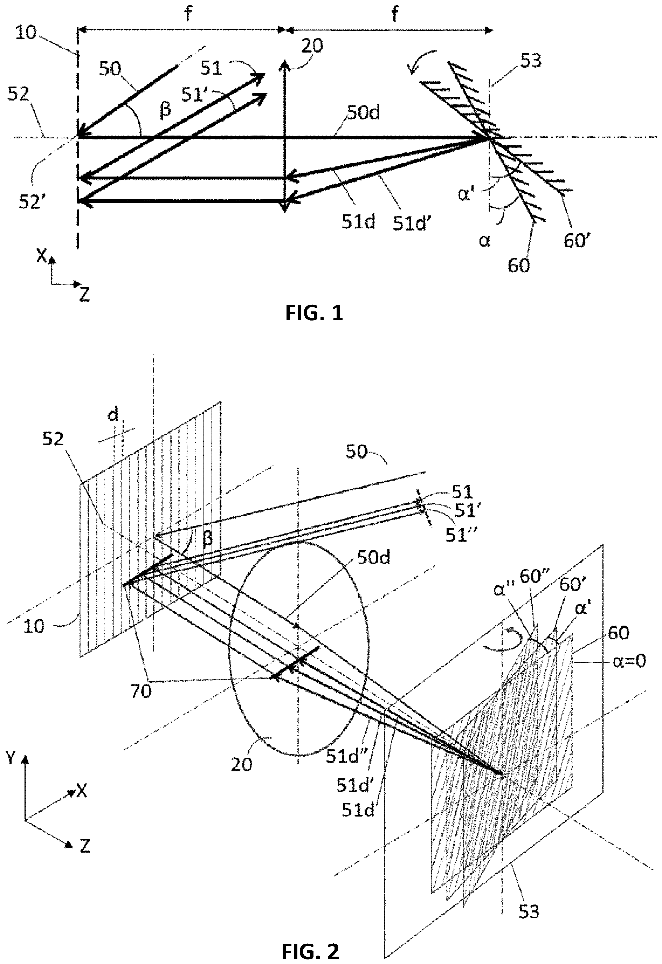

The following description is not to be taken in a limiting sense but is given solely for the purpose of describing the broad principles of the invention. Embodiments of the optical delay line, interferometer and method of the present disclosure will be described by way of example, with reference to the accompanying drawings.
According to the present disclosure, Figure 1 and Figure 2 schematically show, from a top and a perspective view respectively, the main components and working principle of a standard grating-based optical delay line in a single pass configuration that typically belong to the category of the FDODL. As can be observed in Figure 1, the main components of this conventional optical delay line are a diffraction grating 10, a lens 20 and a variable tilt mirror, such as a galvanometric mirror 60 used as scanning component. The galvanometric mirror 60 is rotatable around a Y axis, coinciding with the orientation of the grooves of the diffraction grating 10 and is orthogonal to an optical axis 52 of the lens 20 (which optical axis 52 is parallel to an Z axis according to the XYZ reference used in Figures 1 and 2).
The galvanometric mirror is represented in Figure 1 at two different positions and in Figure 2 at three different positions:

- at a first position the galvanometric mirror 60 is tilted $\alpha$ with respect to a normal plane 53 (parallel to plane XY), being $\alpha$ equal to 0 in Figure 2;
- at a second position the galvanometric mirror 60' is tilted at an angle $\alpha'$ with respect to the same normal plane 53, with $\alpha'>\alpha$;
- at a third position the galvanometric mirror 60" is tilted at a certain angle $\alpha''$ with respect to the same normal plane 53, with $\alpha''> \alpha'$.

The angles $\alpha$, $\alpha'$ and $\alpha''$ represent the degree of tilting of the galvanometric mirror with respect to the plane 53, which is orthogonal to the optical axis 52 of the lens 20.
The diffraction grating 10 (or any other suitable diffractive element) is responsible of diffracting and dispersing a collimated input light beam 50 entering along an input axis 52'. The collimated input light beam 50 is incident on the diffraction grating 10 producing a collimated and dispersed light beam 50d, such that the direction of the diffraction mode of interest is outputted parallel with the optical axis 52 of the lens 20 and at an angle $\beta$ with respect to the input light beam 50.
The standard grating-based optical delay line further comprises an achromatic doublet lens 20 (or any other suitable refractive element), placed in a 1f configuration between the diffraction grating 10 and the galvanometric mirror 60, that is, this lens 20 is located at a distance equal to its focal length f from both components, the diffraction grating 10 and the galvanometric mirror 60. This lens 20 is responsible of focusing each wavelength component of the dispersed light beam 50d after the first incidence of the input light beam 50 on the diffractive element 10, as well as making parallel the chief ray of each wavelength component of the dispersed beam. The lens 20 and therefore its optical axis can be decentred with respect to the dispersed light beam 50d.
When the galvanometric mirror 60 is further tilted with respect to plane 53 and the angle $\alpha$ is changed to $\alpha'$ or $\alpha''$, the dispersed light beam 50d is reflected at different angles and thus redirected towards the lens 20 and the diffraction grating 10 again with a spatial shift with respect to the previous dispersed light beam 50d. Numerical references 51d, 51d', 51d" represent the dispersed light beam 50d after reflection by the galvanometric mirror 60, 60', 60", respectively. Thus, the output light beam 51,51',51" after a second diffraction on the diffraction grating 10 is now a light beam that has been recombined into a broadband beam and is spatially displaced parallelly with respect to the input light beam 50.

The difference in optical path length, OPD, travelled by the input light beam 50 until it is outputted after the second diffraction as the output light beam 51, as $\alpha$ is varied, follows the next equation:

$$OPD = \frac{f \lambda_0 \Delta\alpha}{d \cos(\beta)}$$

where:

    f is the focal distance of the lens 20,
    $\lambda_0$ is the central wavelength of the input light beam 50,
    d is the distance between the grooves of the diffraction grating 10, and
    $\alpha$ and $\beta$ are angles as previously defined.

From the above equation it can be directly concluded that if the tilt angle $\alpha$ of the galvanometric mirror is changed around the Y axis, a variation in the optical path length OPD between the outputted light beam 51, 51', 51" is induced. Actually, the change of the angle $\alpha$, $\alpha$' and $\alpha$" generates a linear displacement 70 in the light beams 51,51', 51", as can be seen in the perspective view shown in Figure 2. This linear displacement 70 (or linear projection of the displacement) is drawn in the lens 20, as well as in the diffraction grating 10, and it is proportional to the optical pathlength range scanned with the standard optical delay line.

However, the high cost of a galvanometric mirror scanner makes its use impractical for low-cost applications.

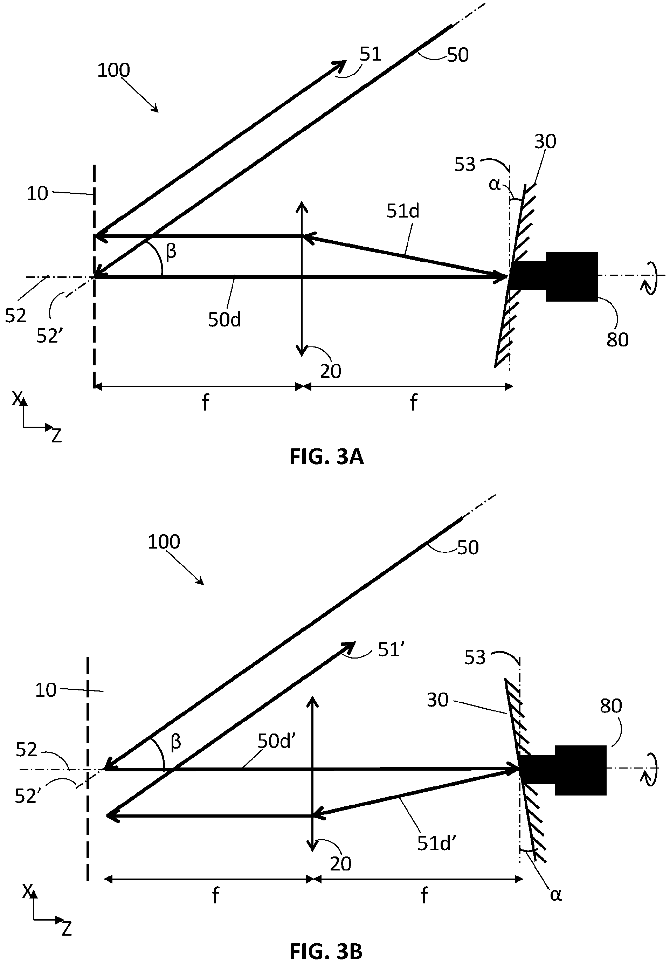

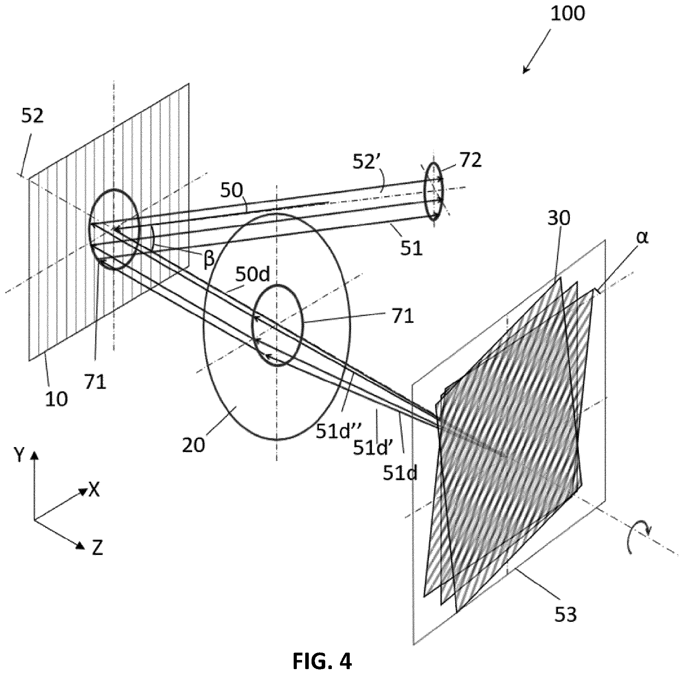

Thus, this disclosure presents a lower-cost and more robust optical delay line 100, which is schematically depicted in a top view in Figures 3A, 3B and in perspective in Figure 4. The same reference numbers as in Figures 1 and 2 have been used in Figures 3A, 3B and 4 for the same or equivalent components or elements (including light beams).

The proposed optical delay line 100 has a diffraction grating 10 and a lens 20, typically an achromatic double lens, and no galvanometric mirror. The optical delay line 100 has a mirror 30 which is tilted at a fixed angle $\alpha$ with respect to the plane 53, orthogonal to the optical axis 52 of the lens 20; and the mirror 30 can be rotated around or parallelly to the optical axis 52 (the optical axis 52 being parallel or collinear to the Z axis according to the XYZ reference used). This mirror 30 is in the following referred to as a spinning tilted mirror 30. This way, in the proposed optical delay line 100 the parallel spatial displacement of the output light beam 51 with respect to the input light beam 50 is generated while rotating the spinning tilted mirror 30 around or parallelly to the optical axis 52, thus inducing a variation of optical path length travelled by the light beam and generating an axial scan.

In the present embodiment, the shaft of a DC or a stepper motor 80 has been coupled to the spinning tilted mirror 30, so as to enable its rotation around the optical axis 52. Other possible simple and robust implementations for rotating the spinning tilted mirror 30 may be provided -even manually- for rotating the spinning tilted mirror 30 around the optical axis.

Between Figure 3A and Figure 3B the spinning tilted mirror 30 has been rotated 180 degrees around the Z axis while the angle $\alpha$ is maintained constant during the axial scan. This way, a symmetric displacement of the output light beam with respect to the input axis 52' is generated between Figure 3A and Figure 3B.

This displacement of the output light beam when the spinning tilted mirror 30 is rotated 360 degrees around the optical axis 52 of the lens 20 can be better seen in Figure 4 where three different rotational positions of the spinning tilted mirror 30 around the optical axis 52 are shown. The dispersed light beam 50d is reflected at the spinning tilted mirror 30 drawing in the lens 20 a footprint of a circle 71, instead of a line as in the standard optical delay line (cf. Fig. 2). The diameter of this circle 71 is determined by the angle $\alpha$ at which the spinning tilted mirror 30 is set, and by the distance between the lens 20 and the spinning tilted mirror 30. Due to the fact that the lens 20 is placed in a 1f configuration to the diffraction grating 10 and to the spinning tilted mirror 30, the chief rays of each wavelength component of the dispersed light beams 51d, 51d' and 51d", after the reflection of the dispersed light beam 50d by the spinning tilted mirror 30 at each rotational position, are focused by the lens 20 generating in the diffraction grating 10 a different spot for each rotational position of the spinning tilted mirror 30, where all the spots generated along the 360 degrees rotation of the spinning tilted mirror 30 generate a footprint of a circle 71 equal to the circle 71 drawn at the lens 20. Finally, after a second incidence on the diffraction grating 10, the output light beam 51 is a recombined broadband collimated light beam which has again been parallelly displaced with respect to the input axis 52'. In the proposed optical delay line 100 an ellipse 72 is described during the axial scan, which is the consequence of the projection at the input axis 52' of the circular footprint 71 generated at the diffraction grating 10, due to the angle $\beta$ between the optical axis 52 and the input axis 52'.

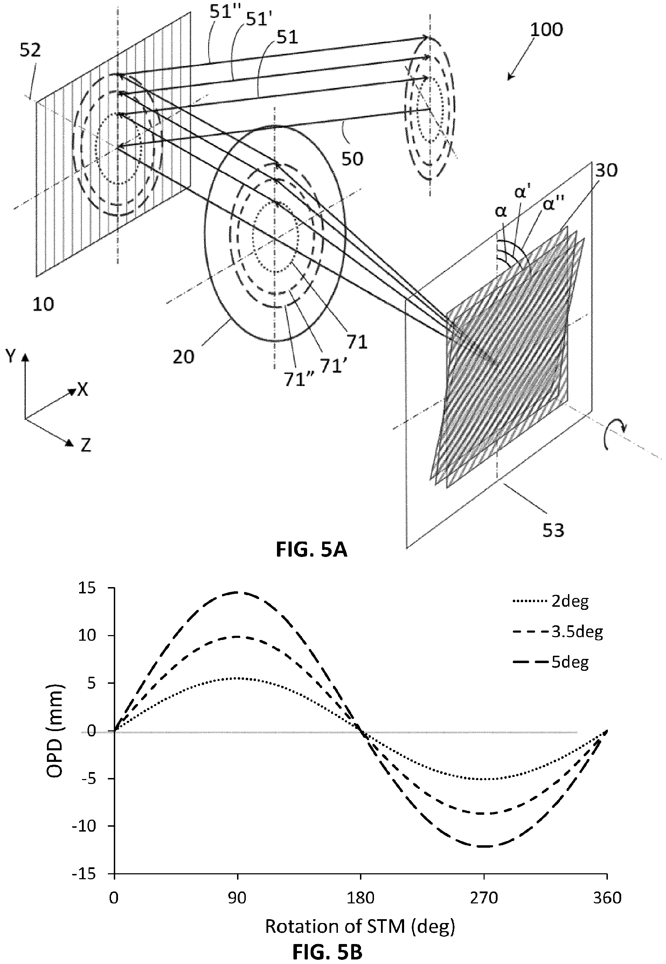

Figure 5A is a schematic perspective view of the optical delay line 100 that intends to show the different displacements induced to the output light beam 51, 51', 51" when three different angles $\alpha$, $\alpha$', $\alpha$" are set to the spinning tilted mirror 30, and then the spinning tilted mirror 30 is rotated with respect to the optical axis 52 of the lens. The different footprints 71, 71', 71" generated at the lens 20 and the diffraction grating 10 when the spinning tilted mirror 30 is rotated 360° around the optical axis 52 are shown. As it can be observed, the diameter of the circular footprint is increased when the angle of tilt $\alpha$ is

increased. This in turn implies an increase in the range of optical path length variation induced by the optical delay line when rotating the spinning tilted mirror 30.

Figure 5B shows the optical path length variation induced at the output light beam during the rotation of the spinning tilted mirror 30, when the spinning tilted mirror 30 is tilted at three different angles $\alpha$, $\alpha'$, $\alpha''$. As it can be observed, the optical pathlength range scanned with the proposed optical delay line is covered twice at each complete rotation of the spinning tilted mirror following a sinusoidal variation.

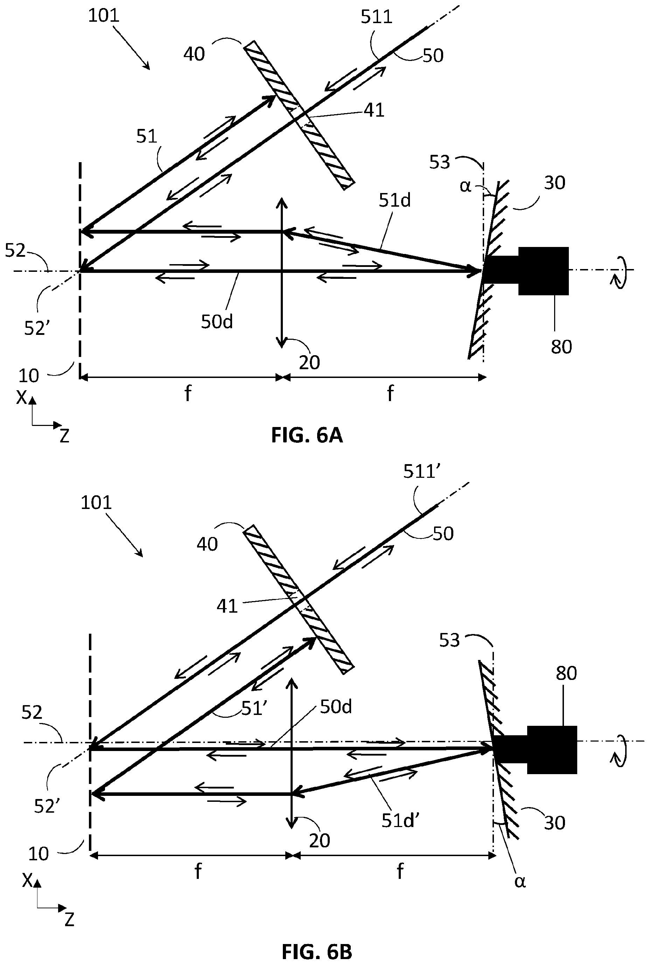

Figures 6A and 6B show top views of another proposed optical delay line 101, also based in a diffractive element, in a double-pass configuration.

The proposed optical delay line 101 is particularly suitable to be used in an interferometric system as the reference arm to perform optical coherence tomography measurements to evaluate, for instance, intraocular distances of a patient's eye. The output light beams 511, 511' should be collinear with the input light beam 50 so that the means for sending the initial collimated light beam, such as a collimator, also serve for receiving the output light beams 511, 511' after having propagated through the optical delay line and thus, having travelled different optical path lengths at each rotational position of the spinning tilted mirror 30. In order to achieve so, the optical delay line 101 comprises a perforated mirror 40 having a hole 41. The perforated mirror 40 has to be placed in a perpendicular manner to the input axis 52' and with its hole 41 centred so as to allow the input light beam 50 and the output light beam 511, 511' to go through it.

Similar to Figures 3A and 3B, Figure 6A and 6B show the optical delay line 101 at two different rotational positions of the spinning tilted mirror 30, the spinning tilted mirror having been rotated 180 degrees around the optical axis 52 of the lens 20 between each other. In both positions, the dispersed light beam 50d is the same, which, after being reflected by the spinning tilted mirror 30 at the two different rotational positions, produces different dispersed light beams 51d, 51d'.

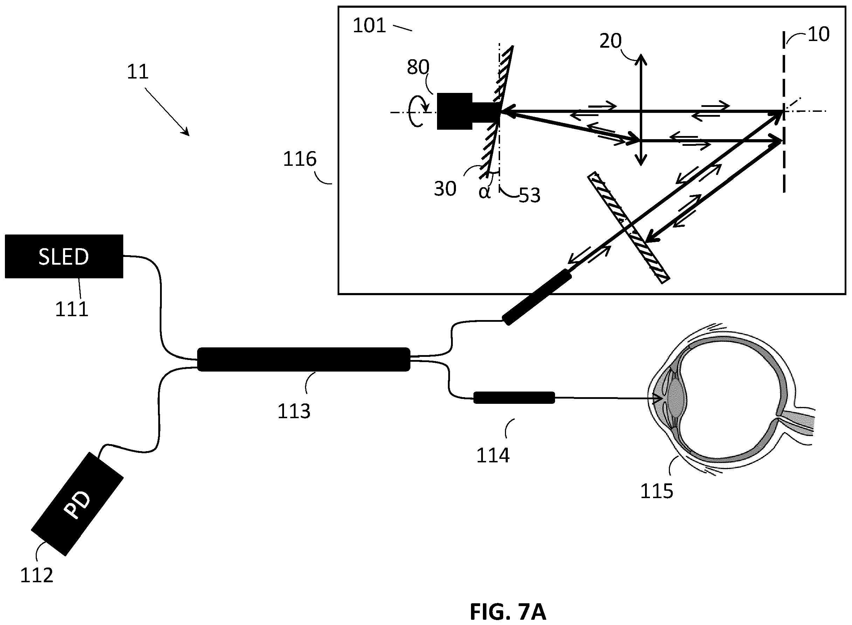

Figure 7A schematically shows an interferometer 11 in a OLCR configuration where the optical delay line 101 of Figures 6A-6B is included in optical communication with the first arm or reference arm 116. The interferometer 11 comprises a light source 111, such as an SLED, a beam splitter 113, a second arm or sample arm 114 with a sample at the end such as an eye 115. The interferometer can be used, for instance, for measuring intraocular distances of the eye 115, such as cornea central thickness, anterior chamber depth, etc. The reference arm 116 ends in the optical delay line 101. The interferometer 11 also includes a photodetector 112 where the interference between the reflected signal from the sample arm 114 and the reference arm 116 is registered to be analysed. The interferogram registered will be dependent on the optical pathlength difference between the sample and the reference arm and thus, while varying the optical pathlength of the reference arm with the mechanism of the optical delay line explained so far, the different ocular surfaces at different depths can be observed.

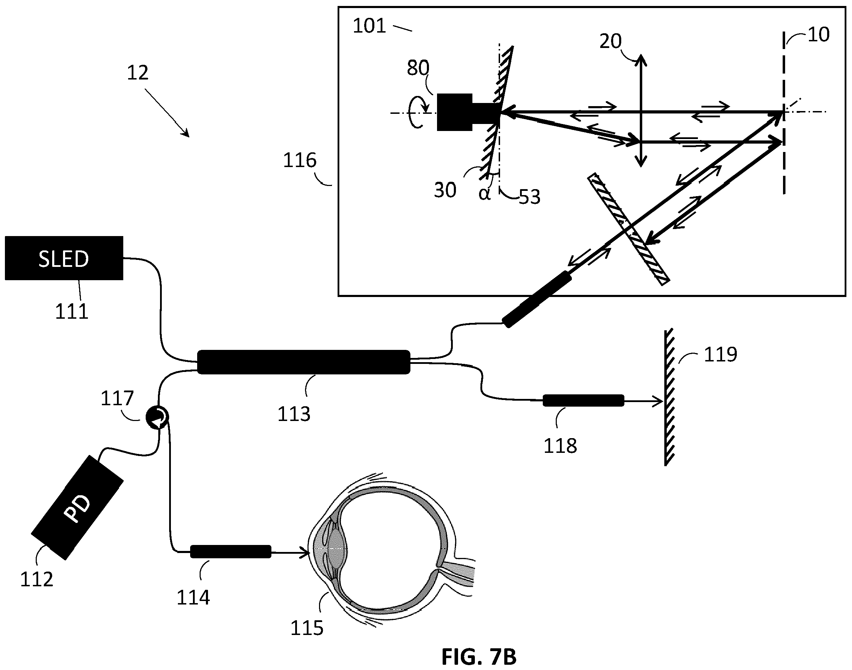

Figure 7B schematically shows a PCI interferometer 12 where the optical delay line 101 of Figures 6A-6B is included in a first arm 116. In this case the interferometer 12 comprises a light source 111, such as an SLED, a beam splitter 113, the first arm 116 with the optical delay line 101, a second arm 118 with a mirror 119, a circulator 117 and a sample arm 114 with a sample at the end such as an eye 115. The interferometer 12 can also be used to measure eye distances. The interferometer 12 also includes a photodetector 112 where the interference between the signal reflected by different eye surfaces is registered to be analysed.

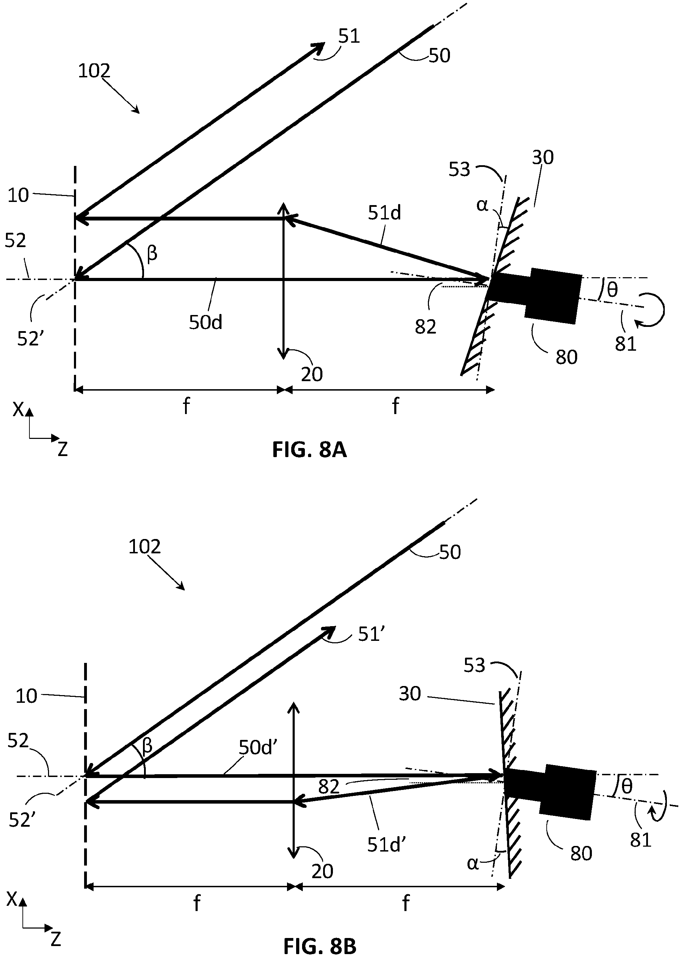

Figures 8A and 8B are schematic top views of another possible configuration of the grating-based optical delay line 102, wherein the axis of rotation 81 of the spinning tilted mirror 30 is now tilted with respect to the optical axis of the refractive element and the centre of rotation of the spinning tilted mirror 30 is at an offset distance 82 with respect to the optical axis of the lens 20. Similarly to Figures 3A-3B, these figures show two different rotational positions of the spinning tilted mirror. Having a mechanical tilt between the axis of rotation 81 of the spinning tilted mirror 30 and the optical axis 52 of the refractive element 20 adds a mechanical complexity for the implementation of the embodiment. Introducing an offset distance 82 between the centre of rotation of the spinning tilted mirror 30 and the optical axis 52 of the lens 20 allows to avoid the use of a phase modulator at the input light beam when the optical delay apparatus explained so far is used at the reference arm as shown in Figure 7.

Thus, the present disclosure solves the shortcomings of the existing solutions with a novel low-cost proposed optical delay line which employs a small mirror and a stepper motor, instead of a galvanometric scanner, thereby providing a robust and inexpensive solution, easy to manufacture. In the proposed solution, the direction normal to the mirror surface is set at a constant tilt angle to the stepper motor shaft direction. This way, the proposed novel low-cost optical delay line consists of effectively spinning a slightly tilted mirror. The scan range and frequency are independently set by the mirror tilt angle and motor speed respectively, unlike in lead screw-based linear mirror translation ODLs or galvanometer mirror-based FDODLs.

The present disclosure is obviously not limited to the specific embodiment(s) described herein, but also encompasses any variations that may be considered by any person skilled in the art (for example, as regards the choice of materials, dimensions, components, configuration, etc.), within the general scope of the disclosure as defined in the claims.

In this text, the terms "comprises", "includes" and its derivations (such as "comprising", "including", etc.) should not be understood in an excluding sense, that is, these terms should not be interpreted as excluding the possibility that what is described and defined may include further elements, steps, etc.

**Claims**

1. An optical delay line (100, 101, 102), comprising a plurality of optical elements in optical communication with each other, wherein:

   - at least one of said plurality of optical elements is a diffractive element (10),
   - at least one of said plurality of optical elements is a reflective element (30),
   - at least one of said plurality of optical elements is a refractive element (20), the refractive element (20) being located between the diffractive element (10) and the reflective element (30);

   wherein the reflective element (30) is tilted at an angle ($\alpha$, $\alpha'$, $\alpha''$) with respect to a plane (53), the reflective element (30) being rotatable around a rotation axis (81, 52) while the angle ($\alpha$, $\alpha'$, $\alpha''$) is maintained, the plane (53) being orthogonal to the rotation axis (81, 52).

2. The optical delay line (100, 101) of claim 1, wherein the diffractive element (10) and the reflective element (30) are located at a focal distance away from the refractive element (20) and on opposite sides.

3. The optical delay line (100, 101) of any of claims 1-2, wherein the rotation axis (81,52) and an optical axis (52) of the refractive element (20) are colinear or parallel to each other.

4. The optical delay line (102) of any of claims 1-2, wherein the rotation axis (81) and an optical axis (52) of the refractive element (20) form a fixed angle ($\theta$).

5. The optical delay line (100, 101, 102) of any previous claim, wherein a centre of rotation of the reflective element (30) has an offset distance (82) with respect to an intersection of the optical axis (52) of the refractive element (20) with a surface of the reflective element (30).

6. The optical delay line (100, 101, 102) of any previous claim, wherein the angle ($\alpha$, $\alpha'$, $\alpha''$) of the reflective element (30) is adjustable such that a range of optical delays can be selected.

7. The optical delay line (100, 101, 102) of any previous claim, further comprising a motor (80) coupled to the reflective element (30), for rotating the reflective element (30) around the rotation axis (81, 52).

8. The optical delay line (101, 102) of any previous claim, further comprising at least another reflective element (40) arranged sequentially after a second incidence (51, 51') of an input light beam (50) on the diffractive element (10) and perpendicular to an input axis (52') of the input light beam (50).

9. The optical delay line (101, 102) of claim 8, wherein the at least another reflective element (40) comprises a through hole.

10. An interferometer (11, 12), the interferometer comprising at least a first arm (116) and a second arm (114), wherein the first arm (116) is arranged in optical communication with an optical delay line (100, 101, 102) according to any of claims 1-9.

11. The interferometer (11, 12) of claim 10, wherein a phase modulator is arranged at/coupled to the first arm (116) before the optical communication with the optical delay line (100, 101, 102).

12. A method for generating an optical delay, the method comprising steps of:

   - receiving an input light beam (50);
   - diffracting the input light beam (50) into a dispersed light beam (50d);
   - focusing each wavelength of the dispersed light beam (50d) into a focused light beam for each wavelength component of the input light beam (50);
   - reflecting, by a reflective element (30), at least a portion of the focused light beam; wherein the step of reflecting is carried out at different angular positions of the reflective element (30) upon rotating the reflective element (30) around a rotation axis (81, 52).

13. The method of claim 12, wherein the reflective element (30) is tilted at an angle ($\alpha$, $\alpha'$, $\alpha''$) with respect to a plane (53) orthogonal to the rotation axis (81, 52), the angle ($\alpha$, $\alpha'$, $\alpha''$) being maintained during rotation of the reflective element (30).

14. The method of any of claims 10-13, wherein the input light beam (50) is subject to phase modulation.

15. The method of any of claims 12-14, wherein the optical delay is produced by an optical delay line according to any of claims 1-9.

FIG. 1

FIG. 2

**FIG. 3A**

**FIG. 3B**

**FIG. 4**

**FIG. 5A**

**FIG. 5B**

**FIG. 6A**

**FIG. 6B**

**FIG. 7A**

**FIG. 7B**

EP 4 374 771 A1

**FIG. 8A**

**FIG. 8B**

Europäisches
Patentamt

European
Patent Office

Office européen
des brevets

# EUROPEAN SEARCH REPORT

**Application Number**

EP 22 38 3130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/036002 A1 (TEARNEY GUILLERMO [US] ET AL) 1 November 2001 (2001-11-01)<br>* paragraph [0006] *<br>* paragraph [0037] – paragraph [0038] *<br>* paragraph [0041] *<br>* paragraph [0057] *<br>* paragraph [0086]; figures 12, 14, 20,21 * | 1–15 | INV.<br>A61B3/10<br>G01B9/02<br>G02B6/28<br>G02B26/00 |
| X | WO 2006/024152 A1 (OTI OPHTHALMIC TECHNOLOGIES [CA]; PODOLEANU ADRIAN GH [GB] ET AL.) 9 March 2006 (2006-03-09)<br>* paragraph [0002] – paragraph [0003] *<br>* paragraph [0007] – paragraph [0008] *<br>* paragraph [0024] – paragraph [0027]; figures 1–7 * | 1–15 | |

TECHNICAL FIELDS
SEARCHED (IPC)

A61B
G01B
G02B

The present search report has been drawn up for all claims

| Place of search | Date of completion of the search | Examiner |
|---|---|---|
| Munich | 19 April 2023 | Horváth, László |

EPO FORM 1503 03.82 (P04C01)

**ANNEX TO THE EUROPEAN SEARCH REPORT
ON EUROPEAN PATENT APPLICATION NO.**

EP 22 38 3130

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2023

| Patent document cited in search report | | Publication date | Patent family member(s) | | Publication date |
|---|---|---|---|---|---|
| US 2001036002 | A1 | 01-11-2001 | US | 6111645 A | 29-08-2000 |
| | | | US | 6282011 B1 | 28-08-2001 |
| | | | US | 2001036002 A1 | 01-11-2001 |
| WO 2006024152 | A1 | 09-03-2006 | EP | 1789832 A1 | 30-05-2007 |
| | | | US | 2006055938 A1 | 16-03-2006 |
| | | | WO | 2006024152 A1 | 09-03-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6111645 A